(19) Europäisches Patentamt
European Patent Office
Office européen des brevets

(11) Publication number: **0 371 559 B1**

# (12) EUROPEAN PATENT SPECIFICATION

(45) Date of publication of patent specification: **12.10.94**

(51) Int. Cl.5: **A61K 31/415**, A61K 31/41, C07D 521/00

(21) Application number: **89203001.6**

(22) Date of filing: **27.11.89**

## (54) Use of benzimidazoles in the treatment of epithelial disorders.

(30) Priority: **29.11.88 US 277152**

(43) Date of publication of application: **06.06.90 Bulletin 90/23**

(45) Publication of the grant of the patent: **12.10.94 Bulletin 94/41**

(84) Designated Contracting States: **AT BE CH DE ES FR GB GR IT LI LU NL SE**

(56) References cited:
**US-A- 4 859 684**

**THERAPEUTIC PROGRESS IN UROLOGICAL CANCERS, vol. 303, 1989, Proceedings of an- International Symposium, Paris, 29th June - 1st July 1988, editors G. P. Murphyet al., pages 205-210, Alan R. Liss, Inc., New York, US; C. MAHLER et al.: "The effects of a new imidazole derivative in advanced prostatic cancer. A preliminary report"**

(73) Proprietor: **JANSSEN PHARMACEUTICA N.V.**
**Turnhoutseweg 30**
**B-2340 Beerse (BE)**

(72) Inventor: **Van Wauwe, Jean P.F.**
**Guido Gezellestraat 61**
**B-2340 Beerse (BE)**
Inventor: **Raeymaekers, Alfons H.M.**
**Aanbeelstraat 1**
**B-2340 Beerse (BE)**

**EP 0 371 559 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Retinoids, in particular retinoic acid and its derivatives, are substances which are known to have a broad spectrum of biological activity. More specifically, these substances affect the differentiation, maintenance and proliferation of various cell types. The ability of retinoids, such as, all-*trans*-retinoic acid, 13-*cis*-retinoic acid, and their derivatives to modulate differentiation in several cell types, whether they are of epithelial or mesenchymal origin, is extensively studied ad reviewed in J. Clin. Chem. Clin. Biochem., 26, 479-488 (1983); Pharmacological Reviews, 36, 935-1005 (1984) ad Arch. Dermatol., 117, 160-180 (1981).

It is known that certain retinoids, particularly the retinoic acids, are used topically for treatment of acne as set forth in U.S. Patent No. 3,729,568. Other known uses of retinoic acid were reviewed in the Journal of America Academy of Dermatology, 4, 505-516 (1981) and the Journal of Medical Chemistry, 25, 1269-1277 (1982) and include, in addition to acne treatment, treatment of senile comedones, nevus comedonicus, linear verrucous nevus, plantar warts, pseudofolliculitis, keratoacanthoma, solar keratosis of extremities, callosities, keratosis palmaris et plantaris, Darier's disease, ichthyosis, psoriasis, acanthosis nigricans, lichen planus, molluscum contagiosum, reactive perforating collagenosis, melasma, corneal epithelial abrasion, Fox-Fordyce disease, cutaneous metastatic melanoma and keloids or hypertrophic scars.

Retinoids such as, all-*trans*-retinoic acid, 13-*cis*-retinoic acid and their derivatives, have also been used in the treatment of carcinomas.

There are however a number of drawback associated with the therapeutic applications of retinoids. The topical applications of retinoids on the one hand often cause significant irritation and peeling due to the relatively high concentrations of retinoid which have to be applied. Systemic applications on the other hand are limited by the toxicity and rapid degradation of the administered retinoids.

The compounds of the invention overcome the problems associated with art known retinoid therapy by suppressing the metabolism of endogenous or exogenously administered retinoic acid.

The present invention concerns the use for the manufacture of a medicament for treating mammals suffering from disorders which are characterized by a increased proliferation and/or abnormal differentiation of epithelial cells of an appropriately substituted benzimidazole which suppresses the plasma elimination of endogenous or exogenously administered retinoic acid. A number of appropriately substituted benzimidazoles are disclosed in our applications U.S. Pat. No 4,859,684 ad EP-A 293,978. Particular compounds for use in the present invention are compounds of formula

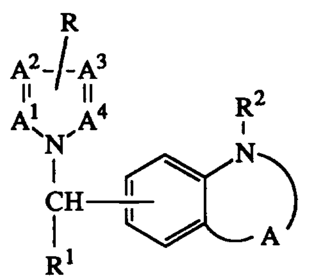

(I)

a pharmaceutically acceptable acid addition salt thereof or a stereochemically isomeric form thereof, wherein

$-A^1=A^2-A^3=A^4-$ is a bivalent radical having the formula

$-CH=N-CH=CH-$ (x);

$-CH=N-CH=N-$ (y);

or

$-CH=N-N=CH-$ (z);

R is hydrogen or $C_{1-6}$alkyl;

$R^1$ is hydrogen; $C_{1-10}$alkyl; $C_{3-7}$cycloalkyl; $Ar^1$ or $Ar^1$-$C_{1-6}$alkyl;

$R^2$ is hydrogen; $C_{3-7}$cycloalkyl; $Ar^1$; $C_{1-10}$alkyl; $C_{1-6}$alkyl substituted with $Ar^1$ or $C_{3-7}$cycloalkyl; hydroxy; $C_{1-10}$alkyloxy; $C_{1-6}$alkyloxy substituted with $Ar^1$ or $C_{3-7}$cycloalkyl; $C_{3-6}$alkenyloxy optionally

substituted with $Ar^2$; $C_{3-6}$alkynyloxy optionally substituted with $Ar^2$; or $Ar^1$-oxy;

A is a bivalent radical having the formula

$-CR^3=N-$ (a)

or

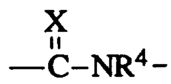 (b),

wherein the carbon atom in the bivalent radical (a) and (b) is connected to - $NR^2$;

said $R^3$ being hydrogen; halo; $C_{1-4}$alkyl substituted with up to 4 halo atoms; $C_{3-7}$cycycloalkyl; $Ar^1$; quinolinyl; indolinyl; $C_{1-10}$alkyl; $C_{1-6}$alkyl substituted with $Ar^1$, $C_{3-7}$cycloalkyl, quinolinyl, indolinyl or hydroxy; $C_{1-10}$alkyloxy; $C_{1-6}$alkyloxy substituted with $Ar^1$ or $C_{3-7}$cycloalkyl; $C_{2-6}$alkenyl optionally substituted with $Ar^1$; $Ar^2$-oxy; $C_{1-6}$alkyloxycarbonyl; carboxyl; $C_{1-6}$alkylcarbonyl; $Ar^1$-carbonyl or $Ar^1$-(CHOH)-;

said X being O or S;

said $R^4$ being hydrogen, $C_{1-6}$alkyl or $Ar^2$-$C_{1-6}$alkyl;

wherein $Ar^1$ is phenyl, substituted phenyl, pyridinyl, aminopyridinyl, imidazolyl, thienyl, halothienyl, furanyl, halofuranyl or thiazolyl; ad $Ar^2$ is phenyl or substituted phenyl; said substituted phenyl in $Ar^1$ ad $Ar^2$ being phenyl substituted with 1,2 or 3 substituents each independently selected from halo, hydroxy, trifluoromethyl, $C_{1-6}$alkyl, $C_{1-6}$alkyloxy, cyano, amino, monk and di($C_{1-6}$alkyl)amino, nitro, carboxyl, formyl and $C_{1-6}$alkyloxycarbonyl.

Preferably said substituted phenyl is phenyl substituted with one or two substituents each independently selected from halo, $C_{1-6}$alkyl, $C_{1-6}$alkyloxy and trifluoromethyl.

As used in the foregoing definitions the term halo is generic to fluoro, chloro, bromo and iodo; the term "$C_{1-6}$alkyl" is meant to include straight chained ad branched saturated hydrocarbon radicals having from 1 to 6 carbon atoms such as, for example, methyl, ethyl, 1-methylethyl, 1,1-dimethylethyl, propyl, 2-methylpropyl, butyl, pentyl, hexyl and the like; "$C_{1-10}$alkyl" is meant to include $C_{1-6}$alkyl radicals, as defined hereinabove, and the higher homologs thereof having from 7 to 10 carbon atoms; the term "$C_{3-7}$cycloakyl" is generic to cyclopropyl, cyclobutyl, cyclopentyl, cyclohexyl and cycloheptyl. "$C_{2-6}$alkenyl" defines straight chained and branched hydrocarbon radicals containing one double bond having from 2 to 6 carbon atoms such as, for example, ethenyl, 2-propenyl, 3-butenyl, 2-butenyl, 2-pentenyl, 3-pentenyl, 3-methyl-2-butenyl and the like; "$C_{2-6}$alkynyl" defines straight chained and branched hydrocarbon radicals containing one triple bond and having from 2 to 6 carbon atoms such as, for example, 2-propynyl, 2-butynyl, 3-butynyl, 2-pentynyl, 3-pentynyl, 4-pentynyl and the like; ad when a $C_{2-6}$alkenyl or a $C_{2-6}$alkynyl is substituted on a heteroatom, then the carbon atom of said $C_{2-6}$alkenyl or said $C_{2-6}$alkynyl connected to said heteroatom preferably is saturated.

It is to be understood that the

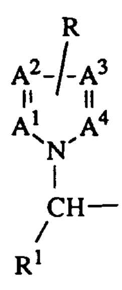

moiety in formula (I) hereinafter referred as the 1H-azol-1-ylmethyl moiety, may be substituted on either the 4, 5, 6 or 7 position of the benzimidazole heterocyclic ring, preferably on the 5 or 6 position, with the 5 position being preferred.

It is evident that the compounds of formula (I) may also contain in their structure a tautomeric system and consequently these compounds can be present in each of their tautomeric forms.

Particular compounds for use in the manufacture of the medicament mentioned hereinabove are those compounds of formula (I) wherein the 1H-azol-1-ylmethyl moiety is substituted on either the 5 or 6 position of the benzimidazole ring; and/or R is hydrogen; and/or $R^1$ is hydrogen; $C_{1-6}$alkyl; $C_{3-7}$cycloalkyl; phenyl;

substituted phenyl; thienyl or furanyl optionally substituted with halo; and/or $R^2$ is hydrogen; $C_{3-7}$cycloalkyl; phenyl; substituted phenyl; pyridinyl; $C_{1-6}$alkyl optionally monosubstituted with phenyl, $C_{3-7}$cycloalkyl, pyridinyl or thienyl; hydroxyl, $C_{1-6}$alkyloxy optionally monosubstituted with phenyl, pyridinyl, thienyl or $C_{3-6}$cycloalkyl; $C_{3-6}$alkenyloxy optionally monosubstituted with phenyl; or $C_{3-6}$alkynyloxy; and/or $R^3$ is hydrogen; $C_{1-4}$alkyl substituted with up to 4 halo atoms; $C_{3-7}$cycloalkyl; phenyl; substituted phenyl; imidazolyl; thiazolyl; thienyl; furanyl; quinolinyl, pyridinyl optionally substituted with amino, $C_{1-10}$alkyl; $C_{1-5}$alkyl optionally monosubstituted with phenyl, pyridinyl, imidazolyl, thienyl, indolyl or hydroxyl; $C_{1-4}$alkyloxy optionally monosubstituted with phenyl; $C_{2-6}$alkenyl optionally monosubstituted with pyridinyl, furanyl, imidazolyl or phenyl; carboxyl; $C_{1-4}$alkyloxycarbonyl; phenylcarbonyl; or hydroxy and phenylmethyl; and/or $R^4$ is hydrogen or phenyl $C_{1-4}$alkyl.

More particular compounds of formula (I) are those particular compounds of formula (I) wherein $R^1$ is hydrogen, $C_{1-6}$alkyl, phenyl, substituted phenyl, thienyl or furanyl; $R^2$ is hydrogen, $C_{1-6}$alkyl, or $C_{1-4}$alkyl substituted with phenyl; $R^3$ is hydrogen, $C_{1-6}$alkyl, phenyl, pyridinyl, $C_{1-6}$alkyl, $C_{1-6}$alkyl monosubstituted with phenyl or $C_{2-6}$alkenyl optionally monosubstituted with furanyl or phenyl; and $R^4$ is hydrogen.

Preferred compounds of formula (I) are those particular compounds of formula (I) wherein $R^1$ is $C_{1-4}$alkyl, phenyl, phenyl substituted with one or two halo, $C_{1-4}$alkyl or $C_{1-4}$alkyloxy substituents, or thienyl; $R^2$ is hydrogen or $C_{1-4}$alkyl; ad $R^3$ is hydrogen or $C_{1-4}$alkyl.

More preferred compounds of formula (I) are those preferred compounds of formula (I) wherein $R^1$ is phenyl or halophenyl, ad $R^2$ and $R^3$ are both independently hydrogen or $C_{1-4}$alkyl.

Most preferred compounds of formula (I) are 5-[(1H-imidazol-1-yl)phenylmethyl]-1H-benzimidazole, (±)-5-[(1H-imidazol-1-yl)phenylmethyl]-2-methyl-1H-benzimidazole, 5-[(1H-imidazol-1-yl)phenylmethyl]-1-methyl-1H-benzimidazole, 5-[1-(1H-imidazole-1-yl)-2-methylpropyl]-2-methyl-1H-benzimidazole, 5-[(3-chlorophenyl) (1H-imidazol-1-yl)methyl]-1H-benzimidazole or (±)-5-[(1H-imidazol-1-yl)phenylmethyl]-2-methyl-1H-benzimidazole, the pharmaceutically acceptable acid addition salts and possible stereoisomers thereof.

The compounds of formula (I) can be prepared by N-alkylating an azole of formula (III) or a alkali metal salt thereof with a benzimidazole of formula (IV).

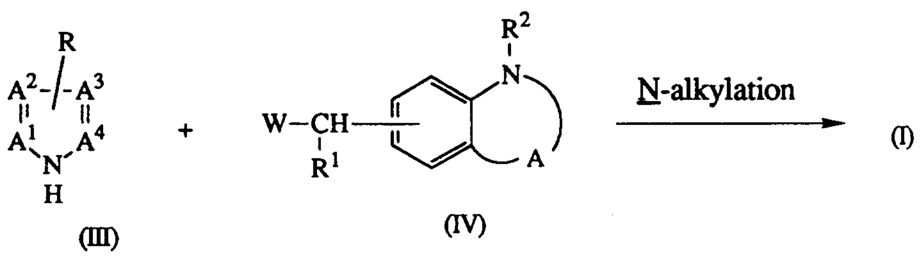


In formula (IV) W represents an appropriate reactive leaving group such as, for example, halo, e.g. fluoro, chloro, bromo, iodo or a sulfonyloxy group, e.g. 4-methylbenzenesulfonyloxy, benzenesulfonyloxy, 2-naphthalenesulfonyloxy, methanesulfonyloxy, trifluoromethanesulfonyloxy and the like reactive leaving groups.

The above described N-alkylations are conveniently carried out by stirring the reactants in the presence of a suitable solvent such as, for example, a aromatic hydrocarbon, e.g., benzene, methylbenzene, dimethylbenzene, and the like; an ester, e.g. ethyl acetate, γ-butyrolacetone and the like; a ketone, e.g. 2-propanone, 4-methyl-2-pentanone and the like; an ether, e.g., 1,4-dioxane, 1,1'-oxybisethane, tetrahydrofuran and the like; a polar aprotic solvent, e.g., N,N-dimethylformamide, N,N-dimethylacetamide, dimethyl sulfoxide, 1-methyl-2-pyrrolidinone, acetonitrile, hexamethylphosphor triamide, 1,3-dimethyl-3,4,5,6-tetrahydro-2(1H)-pyrimidinone, 1,3-dimethyl-2-imidazolidinone, benzonitrile and the like; and mixtures of such solvents. Somewhat elevated temperatures may be appropriate to enhance the rate of the reaction and in some cases the reaction may even be carried out at the reflux temperature of the reaction mixture. The addition of a appropriate base such as, for example, an alkali or a earth alkaline metal carbonate, hydrogen carbonate, hydroxide, amide or hydride, e.g., sodium hydroxide, potassium hydroxide, potassium carbonate, sodium hydride and the like or an organic base, such as, for example, N,N-dimethyl-4-pyridinamine, pyridine, N,N-diethylethanamine or N-(1-methylethyl)-2-propanamine may be employed to pick up the acid which is liberated during the course of the reaction. In some instances it may be advantageous to use a excess of the azole (III) or to convert the azole first into a suitable salt form thereof such as, for example, an alkali or earth alkaline metal salt, by reacting (III) with an appropriate base as

defined hereinabove and subsequently using said salt form in the reaction with the alkylating reagents of formulae (IV). Additionally, it may be advantageous to conduct said N-alkylation reaction under a inert atmosphere such as, for example, oxygen-free argon or nitrogen gas. Said alkylation may also be carried out by applying art-known conditions of phase transfer catalysis reactions.

Compounds of formula (I) wherein $-X^1=X^2-$ is a bivalent radical of formula (x), said compounds being represented by formula (I-x) ad (II-x), may also be prepared by reacting a benzimidazole (IV) with a 1-protected imidazole of formula (III-x) following the N-alkylation procedures described hereinabove for the preparation of compounds of formula (I) starting from (III) and (IV).

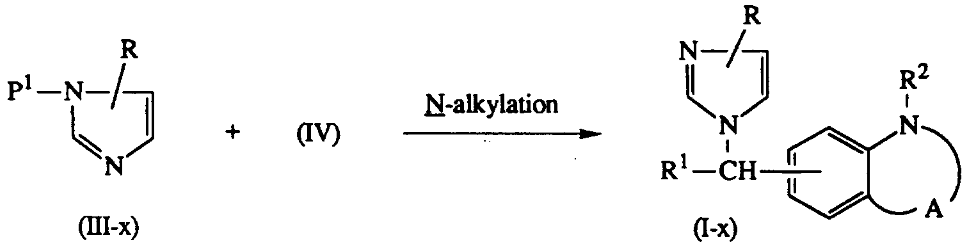


(III-x) + (IV) (I-x)

In (III-x) $P^1$ represents a protective group such as, for example, $C_{1-6}$alkylcarbonyl, $C_{1-6}$alkyloxycarbonyl, arylcarbonyl or a tri($C_{1-6}$alkyl)silyl group. In some instances the reaction of (III-x) with (IV) first yields a 1-protected imidazolium salt of formula (VI-a) which may in situ, or if desired, after isolating and further purifying it, be deprotected by stirring it in an aqueous basic solution or acidic solution.

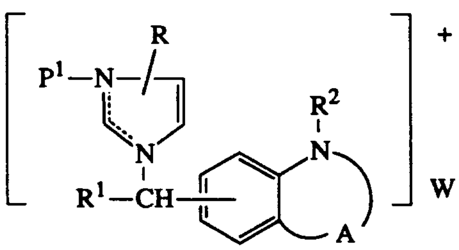

(VI-a)

In (VI-a) $W^-$ is an anion arising from an acid such as, for example, hydrochloric acid, hydrobromic acid, methanesulfonic acid, 4-methylbenzenesulfonic acid and the like acids.

Compounds of formula (I) wherein $-X^1=X^2-$ is a bivalent radical of formula (y), said compounds being represented by formula (I-y), can also be prepared by endo-N-alkylation of a triazolamine of formula (III-y) with a benzimidazole (IV) and subsequent deamination of the thus prepared triazolium salt, wherein $W^-$ is an anion as defined hereinabove.

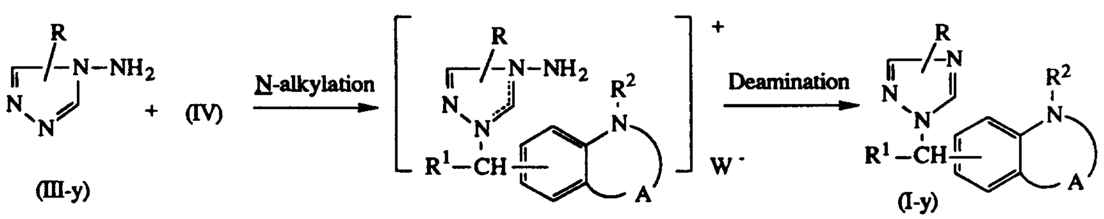


(III-y) + (IV) (I-y)

The endo-N-alkylation reaction of (III-y) with (IV) is carried out according to similar procedures as described hereinabove for the preparation of a compound of formula (I) starting from (III) and (II). Said deamination reaction is conveniently conducted by reaction with an acidic nitrite solution in the presence of an appropriate reductant, or by reaction with an alkylnitrite such as, for example, 1,1-dimethylethylnitrite or isoamylnitrite and the like. Preferably, said deamination reaction is conducted with an aqueous solution of nitrous acid or of a nitrite salt in a suitable acid in the presence of a reducing agent such as, for example, hypophosphorous acid, formic acid, at a lower temperature.

The compounds of formulae (I) may also be prepared by reacting an intermediate of formula (VII) with a reagent of formula (IX) such as, for example, 1,1'-carbonylbis[1H-imidazole].

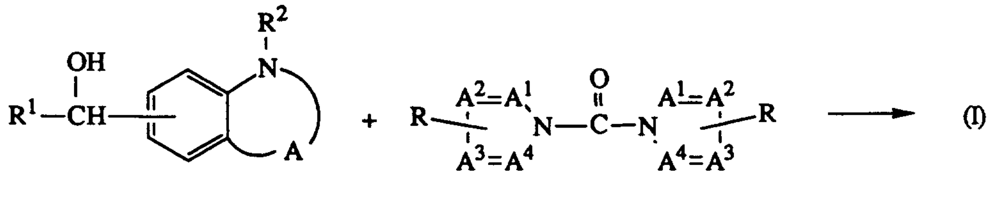

(VII) (IX)

Said reactions may conveniently be conducted in a suitable solvent such as, for example, an ether, e.g., 1,4-dioxane, tetrahydrofuran; a halogenated hydrocarbon, e.g., di-or trichloromethane; a hydrocarbon, e.g., benzene, methylbenzene; N,N-dimethylformamide, N,N-dimethylacetamide, or mixtures of such solvents. In order to enhance the reaction rate, it may be advantageous to heat the reaction mixture.

The compounds of formula (I) may also be prepared by reacting a ketone or aldehyde of formula (X) with an azole (III) in the presence of formic acid or formamides as reducing agents.

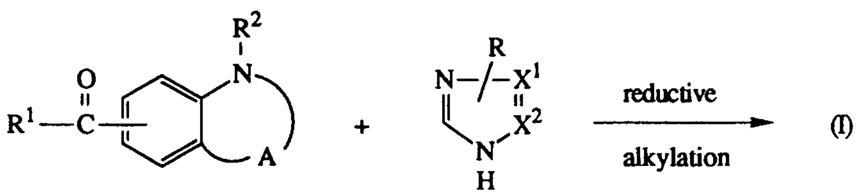


(X) (III)

The compounds of formula (I) can alternatively be prepared according to cyclization procedures outlined in the art for the preparation of benzimidazoles from benzenediamines or ortho nitrobenzeneamines, e.g. U.S. Pat. No. 4,859,684.

For example, benzimidazoles of formula (I) can be prepared by cyclizing an appropriately substituted 1,2-benzenediamine with a carboxylic acid or a functional derivative thereof such as, for example the halide, anhydride, amide and ester form thereof in a suitable acidic medium. The above and similar cyclization procedures for making the compounds of formula (I) are outlined in U.S. Pat. No. 4,859,684.

Alternatively, some compounds of formula (I) may also be prepared according to procedures analogous to those described in the literature for the preparation of azoles by cyclizing an appropriate starting material.

The compounds of formula (I-x) may also be prepared, for example, by cyclizing an intermediate of formula (XII) and desulfurating the thus obtained intermediate of formula (XIV).

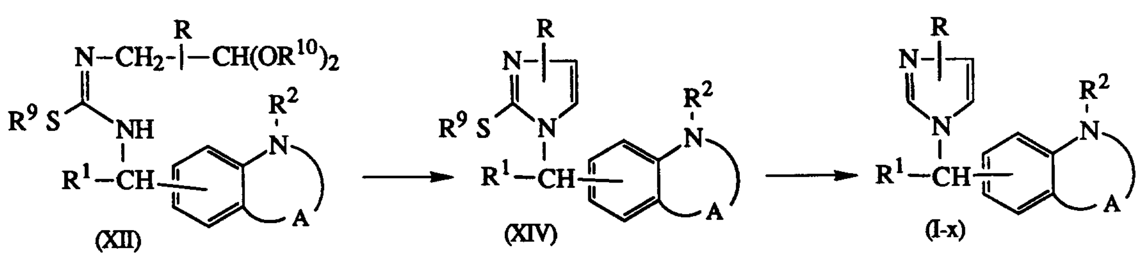

(XII) (XIV) (I-x)

In formulae (XII) and (XIV) $R^9$ represents hydrogen or $C_{1-6}$alkyl and $R^{10}$ represents $C_{1-6}$alkyl or both $R^{10}$ taken together form a $C_{2-3}$alkanediyl radical.

Said cyclization reaction may conveniently be conducted by stirring and heating an intermediate (XII) in an aqueous acidic solvent, e.g. in aqueous hydrochloric or sulfuric acid. The thus obtained intermediate (XIV) may be desulfurated following art-known procedures, e.g., by treatment with Raney nickel in the presence of an alkanol, e.g. methanol, ethanol and the like, or by treatment with nitric acid, optionally in the presence of sodium nitrite.

The compounds of formula (I-y) may be prepared from a hydrazine derivative of formula (XVI) by reaction with s-triazine following the procedures described in J. Org. Chem., 1956, 1037.

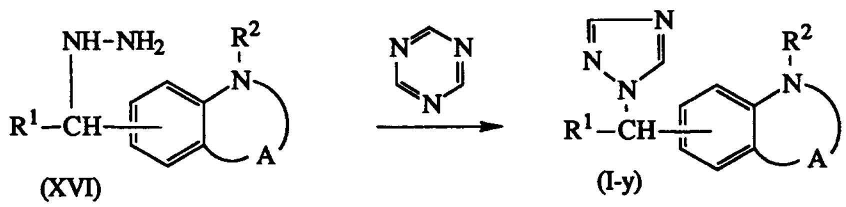

(XVI) (I-y)

The intermediate hydrazine (XVI) and the corresponding intermediate amines may also be converted into azoles, wherein $-A^1=A^2-A^3=A^4-$ is a bivalent radical of formula (x), (y) or (z) following procedures described in U.S. Pat. No. 4,267,179.

The intermediates and the starting materials in the foregoing are known and may be prepared according to art-known methodologies of preparing said or similar compounds. Intermediates and staring compounds in the preparation are specifically described in U.S. Pat. No. 4,859,684. Intermediates of formula (II) are described in EP-A-293,978. A number of such preparation methods will be described hereinafter in more detail.

The intermediate hydrazines (XVI) and amines may conveniently be prepared from a ketone of formula (X) or by reaction with either an acid addition salt thereof, or with hydroxylamine or hydrazine or an acid addition salt or a solvate thereof, and reducing the thus obtained oxime or hydrazone, for example, by catalytic hydrogenation in the presence of hydrogen and an appropriate hydrogenation catalyst, e.g. Raney nickel and the like.

The intermediates of formula (XII) can be prepared from the corresponding amines of formula (XVIII) by reaction with a reagent of formula (XX) and optionally S-alkylating the thus obtained thiourea with a $C_{1-6}$alkylhalide.

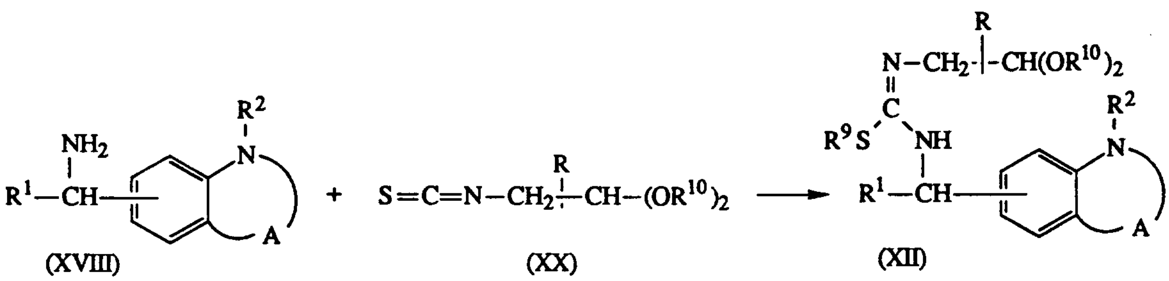

(XVIII) (XX) (XII)

From formulae (I) it is evident that the compounds of this invention may have several asymmetric carbon atoms in their structure. Each of these chiral centers may be present in a R- and a S-configuration, this R- and S-notation being in correspondence with the rules described by R.S. Cahn, C. Ingold and V. Prelog in Angew. Chem., Int. Ed. Engl., 5, 385, 511 (1966).

Pure stereochemically isomeric forms of the compounds of formulae (I) may be obtained by the application of art-known procedures. Diastereoisomers may be separated by physical separation methods such as selective cyrstallization and chromatographic techniques, e.g., counter current distribution, and enantiomers may be separated from each other by the selective crystallization of their diastereomeric salts with optically active acids.

Pure stereochemically isomeric forms may also be derived from the corresponding pure stereochemically isomeric forms of the appropriate starting materials, provided that the reaction occurs stereospecifically.

Stereochemically isomeric forms of the compounds of formulae (I) are naturally intended to be embraced within the scope of the invention.

An additional feature of the invention comprises the fact that those compounds of formula (I) wherein $-A^1=A^2-A^3=A^4-$ is a bivalent radical of formula (y) or (z), or wherein $-A^1=A^2-A^3=A^4-$ is a bivalent radical of formula (x) with R being $C_{1-6}$alkyl, the pharmaceutically acceptable acid addition salts thereof and the stereochemically isomeric forms thereof, are novel compounds.

Particular novel compounds are those novel compounds wherein $-A^1=A^2-A^3=A^4-$is a bivalent radical of formula (y) or (z).

Preferred novel compounds within the invention are those particular novel compounds wherein $-A^1=A^2-A^3=A^4-$ is a bivalent radical of formula (y); an/or $R^1$ is phenyl or halophenyl; and/or $R^2$ is hydrogen or $C_{1-4}$alkyl; and/or $R^3$ is hydrogen or $C_{1-4}$alkyl.

Some of the compounds of formula (I) which can be used as active ingredient in the compositions and methods of treatment according to the present invention are listed in the following tables with the purpose of illustrating the invention.

Table 1

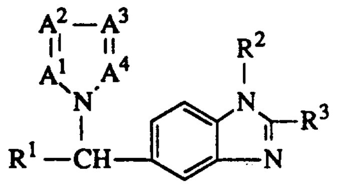

| Comp No. | p* | $-A^1=A^2-A^3=A^4-$ | $R^1$ | $R^2$ | $R^3$ | mp.(°C)/ salt |
|---|---|---|---|---|---|---|
| 1 | 5 | -CH=CH-N=CH- | $C_6H_5$- | H- | H- | 186.2 |
| 2 | 5 | -CH=CH-N=CH- | $C_6H_5$- | H- | $CH_3$- | 118.4 |
| 3 | 5 | -CH=CH-N=CH- | 2-thienyl- | H- | H- | 101.0 |
| 4 | 5 | -CH=CH-N=CH- | 2-thienyl- | H- | $CH_3$- | 108.9 |
| 5 | 5 | -CH=CH-N=CH- | 4-F-$C_6H_4$- | H- | $CH_3$- | 110.6 |
| 6 | 5 | -CH=CH-N=CH- | 2,4-$(Cl)_2$-$C_6H_3$- | H- | $CH_3$- | 138.4 |
| 7 | 5 | -CH=CH-N=CH- | 3-Cl-$C_6H_4$- | H- | $CH_3$- | 113.3 |
| 8 | 5 | -CH=CH-N=CH- | 3-$CH_3$-$C_6H_4$- | H- | H- | 104.8 |

| Comp No. | p* | $-A^1=A^2-A^3=A^4-$ | $R^1$ | $R^2$ | $R^3$ | mp.(°C)/ salt |
|---|---|---|---|---|---|---|
| 9 | 5 | -CH=CH-N=CH- | $C_6H_5$- | H- | 2-pyridyl- | 123.3 |
| 10 | 5 | -CH=CH-N=CH- | c.$C_3H_5$- | H- | H- | 73.5 |
| 11 | 5 | -CH=CH-N=CH- | $C_6H_5$- | H- | 3-pyridiyl- | 133.1 |
| 12 | 6 | -N=CH-N=CH- | H- | OH- | 3-pyridiyl- | 219.3 |
| 13 | 5 | -CH=CH-N=CH- | $C_6H_5$- | H- | $C_6H_5$- | 134.5 |
| 14 | 6 | -N=CH-N=CH- | H- | $OCH_3$- | 3-pyridiyl- | 141.5 |
| 15 | 5 | -N=CH-N=CH- | H- | H- | $CH_3$- | 241.0/ 2HCl |
| 16 | 5 | -N=CH-N=CH- | H- | H- | H- | 184.4 |
| 17 | 5 | -N=CH-N=CH- | H- | H- | $C_6H_5$- | 239.7 |
| 18 | 5 | -N=CH-N=CH- | H- | H- | 3-pyridiyl- | 222.5 |
| 19 | 5 | -CH=CH-N=CH- | $C_6H_5$- | H- | $C_6H_5$-$CH_2$- | 189.9 |
| 20 | 5 | -CH=CH-N=CH- | $C_4H_9$- | H- | $CH_3$- | - |
| 21 | 5 | -CH=CH-N=CH- | $C_6H_5$- | $CH_3$- | H- | 138.7 |
| 22 | 5 | -CH=CH-N=CH- | i-$C_3H_7$- | H- | $CH_3$- | 214.8/2HCl/$H_2O$ |
| 23 | 5 | -CH=CH-N=CH- | $C_6H_5$- | H- | 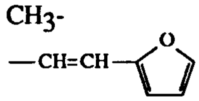 | 134.7 (E) |
| 24 | 5 | -CH=CH-N=CH- | $C_3H_7$- | H- | $CH_3$- | 174.2/1.5$(COOH)_2$ |
| 25 | 5 | -CH=CH-N=CH- | $C_6H_5$- | H- | 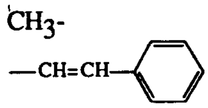 | 140.6 (E) |
| 26 | 5 | -CH=CH-N=CH- | 2-furanyl- | H- | H- | 150.9 |
| 27 | 5 | -CH=CH-N=CH- | $C_6H_5$- | H- | $CH_3$- | 77.2/$H_2O$/** |
| 28 | 5 | -CH=CH-N=CH- | 3-Cl-$C_6H_4$- | H- | H- | 200.2/HCl |
| 29 | 5 | -CH=CH-N=CH- | 3-Cl-$C_6H_4$- | $CH_3$- | H- | 131.2/1/2$H_2O$ |
| 30 | 5 | -CH=CH-N=CH- | 3-Cl-$C_6H_4$- | $C_6H_5$-$CH_2$- | H- | 59.6/1/2EtOH |
| 31 | 5 | -N=CH-N=CH- | 3-Cl-$C_6H_4$- | H- | $CH_3$- | 205.4/2$(COOH)_2$ |
| 32 | 5 | -N=CH-N=CH- | 3-Cl-$C_6H_4$- | H- | H- | 210.0 |
| 33 | 5 | -CH=CH-N=CH- | $C_6H_5$- | H- | $CH_3$- | 128.5 / $H_2O$ |

* : p indicates the position of the 1H-azol-1-ylmethylmoiety on the benzimidazole ring
** = $[\alpha]_D$= -29.57° (c = 0.5% in methanol)

The use of the compounds of formula (I), their pharmaceutically acceptable acid addition salts and their possible stereochemically isomeric forms for the manufacture of a medicament as mentioned hereinabove is based on their useful property to delay the metabolism of retinoids, such as, all-*trans*-retinoic acid, 13-*cis*-retinoic acid and their derivatives. The latter results in more sustained/higher tissue concentrations of retinoids and improved control of differentiation and growth of various cell types. Said property to delay the metabolism of retinoids can easily be evidenced in various in *vivo* experiments. A particular test procedure is described hereinafter as the "Metabolism of endogenous or exogenously administered all-*trans*-retinoic acid"-test. As such, the compounds of formula (I) can be used to control the rate of growth and differentiation of normal, preneoplastic and neoplastic epithelial cells. The ability of retinoids, such as, 13-*cis*-retinoic acid, all-*trans*-retinoic acid and their derivatives to modulate differentiation and proliferation in several cell types is extensively studied and reviewed in J. Clin. Chem. Clin, Biochem., 26, 479-488 (1983);

Pharmacological Reviews 36, 935-1005, (1984), Arch. Dermatol. 117, 160-180; (1981) and Journal of Medicinal Chemistry 25, 1269-1277, (1982).

The compounds of formulae (I), their pharmaceutically acceptable acid addition salts and their possible stereochemically isomeric forms are therefore useful to treat disorders which are characterized by an increased proliferation and/or abnormal differentiation of epithelial cells, in particular cells of which the growth and differentiation is sensitive to the actions of retinoids. Other uses include the ability to cure and/or reduce a variety of disorders of keratinization such as, for example, acne, psoriasis, lamellar ichthyosis, plantar warts, callosities, acanthosis nigricans, lichen planus, molluscum, melasma, corneal epithelial abrasion, geographic tongue, Fox-Fordyce disease, cutaneous metastatic melanoma and keloids, epidermolytic hyperkeratosis, Darier's disease, pityriasis rubra pilaris, congenital ichthyosiform erythroderma, hyperkeratosis palmaris et plantaris, and similar disorders.

The anti-tumor activity, especially in retinoic acid sensitive tumors, may be demonstrated in several retinoic acid-sensitive cell lines and solid tumors such as, for example, in Ta3-Ha induced mamma tumors in female mice.

Those of skill in treating disorders which are characterized by an excessive proliferation and/or abnormal differentiation of tissues could determine the effective amount from the test results presented hereinafter. In general it is contemplated than an effective amount would be from 0.001 mg/kg to 50 mg/kg body weight and more preferably from 0.01 mg/kg to 10 mg/kg body weight.

The active ingredients of formulae (I) are most preferably applied in the form of appropriate compositions. As appropriate compositions there may be cited all compositions usually employed for systemically or topically administering drugs. To prepare the pharmaceutical compositions of this invention, an effective amount of the particular compound, optionally in acid-addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules, and tablets. Because of their ease in administration, tablets and capsules represents the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositons suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

As appropriate compositions for topical application there may be cited all compositions usually employed for topically administering drugs, e.g., creams, gellies, dressings, shampoos, tinctures, pastes, ointments, salves, powders, liquid or semi-liquid formulation and the like. Application of said compositions may be by aerosol, e.g. with a propellent such as nitrogen, carbon dioxide, a freon, or without a propellent such as a pump spray, drops, lotions, or a semisolid such as a thickened composition which can be applied by a swab. In particular compositions, semisold compositions such as salves, creams, pastes, gellies, ointments and the like will conveniently be used.

It is especially advantageous to formulate the aforementioned pharmaceutical compositions in dosage unit form for ease of administration and uniformity of dosage. Dosage unit form as used in the specification and claims herein refers to physically discreate units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powders packets, wafers, injectable solutions or suspensions, teaspoonfuls, tablespoonfuls and the like, and segregated multiples thereof.

Other such compositions are preparations of the cosmetic type, such as toilet waters, packs, lotions, skin milks or milky lotions. Said preparations contain, besides the active ingredient of formula (I), components usually employed in such preparations. Examples of such components are oils, fats, waxes, surfactants, humectants, thickening agents, antioxidants, viscosity stabilizers, chelating agents, buffers,

preservatives, perfumes, dyestuffs, lower alkanols, and the like. If desired, further ingredients may be incorporated in the compositions, e.g. antiinflammatory agents, antibacterials, antifungals, disinfectants, vitamins, sunscreens, antibiotics, or other anti-acne agents.

Examples of oils comprise fats and oils such as olive oil and hydrogenated oils; waxes such as beeswax and lanolin; hydrocarbons such as liquid paraffin, ceresin, and squalane; fatty acids such as stearic acid and oleic acid; alcohols such as cetyl alcohol, stearyl alcohol, lanolin alcohol, and hexadecanol; and esters such as isopropyl myristate, isopropyl palmitate and butyl stearate. As examples of surfactants there may be cited anionic surfactants such as sodium stearate, sodium cetylsulfate, polyoxyethylene laurylether phosphate, sodium N-acyl glutamate; cationic surfactants such as stearyldimethylbenzylammonium chloride and stearyltrimethylammonium chloride; ampholytic surfactants such as alkylaminoethylglycine hydrochloride solutions and lecithin; and nonionic surfactants such as glycerin monostearate, sorbitan monostearate, sucrose fatty acid esters, propylene glycol monostearate, polyoxyethylene oleylether, polyethylene glycol monostearate, polyoxyethylene sorbitan monopalmitate, polyoxyethylene coconut fatty acid monoethanolamide, polyoxyethylene polyoxypropylene glycol (e.g. the materials sold under the trademark "Pluronic"), polyoxyethylene castor oil, and polyoxyethylene lanolin. Examples of humectants include glycerin, 1,3-butylene glycol, and propylene glycol; examples of lower alcohols include ethanol and isopropanol; examples of thickening agents include xanthan gum, hydroxypropyl cellulose, hydroxypropyl methyl cellulose, polyethylene glycol and sodium carboxymethyl cellulose; examples of antioxidants comprise butylated hydroxytoluene, butylated hydroxyanisole, propyl gallate, citric acid and ethoxyquin; examples of chelating agents include disodium edetate and ethanehydroxy diphosphate; examples of buffers comprise citric acid, sodium citrate, boric acid, borax, and disodium hydrogen phosphate; and examples of preservatives are methyl parahydroxybenzoate, ethyl parahydroxybenzoate, dehydroacetic acid, salicylic acid and benzoic acid.

For preparing ointments, creams, toilet waters, skin milks, and the like, typically from 0.01 to 10% in particular from 0.1 to 5% and more in particular from 0.2 to 2.5% of the active ingredient of formula (I) will be incorporated in said compositions. In ointments or creams, the carrier for example consists of 1 to 20%, in particular 5 to 15% of a humectant, 0.1 to 10% in particular from 0.5 to 5% of a thickener and water; or said carrier may consist of 70 to 99%, in particular 20 to 95% of a surfactant, and 0 to 20%, in particular 2.5 to 15% of a fat; or 80 to 99.9% in particular 90 to 99% of a thickener; or 5 to 15% of a surfactant, 2-15% of a humectant, 0 to 80% of an oil, very small (<2%) amounts of preservative, colouring agent and/or perfume, and water. In a toilet water, the carrier for example consists of 2 to 10% of a lower alcohol, 0.1 to 10% or in particular 0.5 to 1% of a surfactant, 1 to 20%, in particular 3 to 7% of a humectant, 0 to 5% of a buffer, water and small amounts (<2%) of preservative, dyestuff and/or perfume. In a skin milk, the carrier typically consists of 10-50% of oil, 1 to 10% of surfactant, 50-80% of water and 0 to 3% of preservative and/or perfume. In the aforementioned preparations, all % symbols refer to weight by weight percentage. The humectant, surfactant, oil, etc... referred to in said preparations may be any such component used in the cosmetic arts but preferably will be one or more of the components mentioned hereinabove. Further, when in the above compositions one or more of the components make up the major part of the composition, the other ingredients can evidently be not present at their indicated maximum concentration and therefore will make up the remainder of the composition.

Particular compositions for use in the method of the present invention are those wherein the active ingredient of formula (I) is formulated in liposome-containing compositions. Liposomes are artificial vesicles formed by amphiphatic molecules such as polar lipids, for example, phosphatidyl cholines, ethanolamines and serines, sphingomyelins, cardiolipins, plasmalogens, phosphatidic acids and cerebiosides. Liposomes are formed when suitable amphiphathic molecules are allowed to swell in water or aqueous solutions to form liquid crystals usually of multilayer structure comprised of many bilayers separated from each other by aqueous material (also referred to as coarse liposomes). Another type of liposome known to be consisting of a single bilayer encapsulating aqueous material is referred to as a unilamellar vesicle. If water-soluble materials are included in the aqueous phase during the swelling of the lipids they become entrapped in the aqueous layer between the lipid bilayers.

Water-soluble active ingredients such as, for example, most of the salt forms of the compound of formula (I) are encapsulated in the aqueous spaces between the molecular layers. The lipid soluble active ingredient of formula (I) is predominantly incorporated into the lipid layers, although polar head groups may protrude from the layer into the aqueous space. The encapsulation of these compounds can be achieved by a number of methods. The method most commonly used involves casting a thin film of phospholipid onto the walls of a flask by evaporation from an organic solvent. When this film is dispersed in a suitable aqueous medium, multilamellar liposomes are formed. Upon suitable sonication, the coarse liposomes form smaller similarly closed vesicles.

Water-soluble active ingredients are usually incorporated by dispersing the cast film with an aqueous solution of the compound. The unencapsulated compound is then removed by centrifugation, chromatography, dialysis or other art-known suitable procedures. The lipid-soluble active ingredient is usually incorporated by dissolving it in the organic solvent with the phospholipid prior to casting the film. If the solubility of the material in the lipid phase is not exceeded or the amount present is not in excess of that which can be bound to the lipid, liposomes prepared by the above method usually contain most of the material bound in the lipid bilayers; separation of the liposomes from unencapsulated material is not required.

A particularly convenient method for preparing liposome formulated forms of the active ingredient of formula (I) is the method described in EP-A-253,619, incorporated herein by reference. In this method, single bilayered liposomes containing encapsulated active ingredients are prepared by dissolving the lipid component in an organic medium, injecting the organic solution of the lipid component under pressure into an aqueous component while simultaneously mixing the organic and aqueous components with a high speed homogenizer or mixing means, whereupon the liposomes are formed spontaneously.

The single bilayered liposomes containing the encapsulated active ingredient of formula (I) can be employed directly or they can be employed in a suitable pharmaceutically acceptable carrier for topical administration. The viscosity of the liposomes can be increased by the addition of one or more suitable thickening agents such as, for example xanthan gum, hydroxypropyl cellulose, hydroxypropyl methylcellulose and mixtures thereof. The aqueous component may consist of water alone or it may contain electrolytes, buffered systems and other ingredients, such as, for example, preservatives. Suitable electrolytes which can be employed include metal salts such as alkali metal and alkaline earth metal salts. The preferred metal salts are calcium chloride, sodium chloride and potassium chloride. The concentration of the electrolyte may vary from zero to 260 mM, preferably from 5 mM to 160 mM. The aqueous component is placed in a suitable vessel which can be adapted to effect homogenization by effecting great turbulence during the injection of the organic component. Homogenization of the two components can be accomplished within the vessel, or, alternatively, the aqueous and organic components may be injected separately into a mixing means which is located outside the vessel. In the latter case, the liposomes are formed in the mixing means and then transferred to another vessel for collection purpose.

The organic component consists of a suitable non-toxic, pharmaceutically acceptable solvent such as, for example ethanol, glycerol, propylene glycol and polyethylene glycol, and a suitable phospholipid which is soluble in the solvent. Suitable phospholipids which can be employed include lecithin, phosphatidylcholine, phosphatidylethanolamine, phosphatydylserine, phosphatidylinositol, lysophosphatidylcholine and phosphatidyl glycerol, for example. Other lipophilic additives may be employed in order to selectively modify the characteristics of the liposomes. Examples of such other additives include stearylamine, phosphatidic acid, tocopherol, cholesterol and lanolin extracts.

It may be advantageous to use micronized forms of the active ingredient of formula (I), i.e., material having an average particle size of less than 10 microns, as the high surface area will facilitate the dissolution of the liposomal components.

In addition, other ingredients which can prevent oxidation of the phospholipids may be added to the organic component. Examples of such other ingredients include tocopherol, butylated hydroxyanisole, butylated hydroxytoluene, ascorbyl palmitate and ascorbyl oleate. Preservatives such as benzoic acid, methyl paraben and propyl paraben may also be added.

The liposome-formulated forms of the active ingredient of formula (I), particularly those obtained in the above-referred method of preparing such liposome formulated forms, may be used as such or in combination with any of the aforementioned carriers to prepare ointments, creams, gelées, toilet waters, etc...

Apart from the above-described compositions, use may be made of covers, e.g. plasters, bandages, dressings, gauze pads and the like, containing an appropriate amount of a composition as referred hereinabove. In some cases use may be made of plasters, bandages, dressings, gauze pads and the like which have been impregnated or sprinkled with a liquid formulation containing the active agent, e.g. with an aseptic aqueous solution, or strewn with a powdery solid composition, or smeared, covered or coated with a semi-liquid composition.

The following examples are intended to illustrate the scope of the present invention.

## Experimental part

A. Preparation of the compounds

Example 1

A mixture of 29.4 parts of 5-[chloro(3-chlorophenyl)methyl]-2-methyl-1H-benzimidazole monohydrochloride, 18.6 parts of 1H-1,2,3-triazole and 240 parts of acetonitrile was stirred for 3 hours at reflux temperature. After evaporation to dry, the residue was taken up in water and treated with potassium carbonate. The product was extracted three times with 39 parts of dichloromethane. The combined extracts were dried, filtered and evaporated to dry. The residue was purified by column chromatography over silica gel using a mixture of dichloromethane ad methanol (95:5 by volume) as eluent. The pure fractions were collected and the eluent was evaporated. The residue was converted into the ethanedioate salt in ethanol. The salt was filtered off and recrystallized from a mixture of ethanol and 2-propanone. The product was filtered off and dried, yielding 6.3 parts (14.0%) of 5-[(3-chlorophenyl)(1H-1,2,3-triazol-1-yl)methyl]-2-methyl-1H-benzimidazole ethanedioate(1:2); mp. 205.4 °C (comp.31).

Example 2

A mixture of 5.6 parts of 1-methyl-α-phenyl-1H-benzimidazole-5-methanol, 4.05 parts of 1,1'-carbonylbis[1H-imidazole] and 54 parts tetrahydrofuran was stirred for 4 hours at reflux temperature. The tetrahydrofuran layer was evaporated and water was added to the residue. The decanted oil was dissolved in dichloromethane. The organic layer was dried, filtered and evaporated. The residue was purified by column chromatography over silica gel using a mixture of trichloromethane, methanol and methanol, saturated with ammonia, (90:5:5 by volume) as eluent. The pure fractions were collected and the eluent was evaporated. The residue was further purified by column chromatography over silica gel using a mixture of trichloromethane and methanol (93:7 by volume) as eluent. The pure fractions were collected and the eluent was evaporated. The residue was washed with 2,2'-oxybispropane and dried, yielding 2.9 parts (42.9%) of 5-[(1H-imidazol-1-yl)phenylmethyl]-1-methyl-1H-benzimidazole; mp. 138.7 °C (comp. 21).

Example 3

A mixture of 6.2 parts of 4-[1-(1H-imidazol-1-yl)-2-methylpropyl]-1,2-benzenediamine, 6.5 parts of ethyl ethanimidate hydrochloride and 80 parts of ethanol was stirred for 3 hours at reflux temperature. After evaporation to dry, the residue was taken up in water and sodium carbonate. The product was extracted three times with 120 parts of trichloromethane. The combined extracts were dried, filtered and evaporated. The residue was purified by column chromatography over silica gel using a mixture of trichloromethane and methanol (95:5 by volume) as eluent. The pure fractions were collected and the eluent was evaporated. The residue was converted into the hydrochloride salt in a mixture of 2-propanone and ethanol. The salt was filtered off and crystallized from a mixture of ethanol and 2-propanone. The product was filtered off and dried, yielding 4 parts (44%) of 5-[1-(1H-imidazol-1yl)-2-methylpropyl]-2-methyl-1H-benzimidazole dihydrochloride.monohydrate; mp. 214.8 °C (comp. 22).

All other compounds listed in Table I can be obtained by analogous methods of preparation.

B. Pharmaceutical Examples

Example 4

Metabolism of exogenously administered all-*trans*-retinoic acid

Male Wistar rats weighing 200~210 g were orally treated with vehicle (PEG 200) or with 40 mg/kg of a compound of formula (I). One hour later, the animals were anesthetized with ether and injected intrajugularly with 0.50 ml saline solution containing 20 µg of all-*trans*-retinoic acid. Two hours after this injection, rats were killed by decapitation and blood was collected on heparin. Blood samples were centrifuged (1000 g, 15 min) and plasma was recovered to determine the quantity of plasmatic all-*trans*-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 1, 2, 3, 4, 5, 6, 7, 8, 9, 11, 13, 19, 20, 21, 22, 24, 28, 29, 30, 33 and 34 enhanced the recovery of all-*trans*-retinoic acid from the plasma to a least 8 ng/ml after dosing with 40 mg/kg.

Example 5

Metabolism of endogenous all-*trans*-retinoic acid

Male Wistar rats weighing 200~210 g were orally treated with vehicle (PEG 200) or with 40 mg/kg of a compound of formula (I). Two hours after drug administration, the rats were killed by decapitation and blood was collected on heparin. Blood samples were centrifuged (1000 g, 15 min) and plasma was recovered to determine the quantity of plasmatic all-*trans*-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 1, 2, 7, 13, 21, 22, 27, 28 and 33 enhanced the recovery of all-*trans*-retinoic acid from the plasma to a least 1 ng/ml.

C) Composition Examples

The following formulations exemplify typical pharmaceutical and cosmetical compositions in dosage unit form suitable for systemic or topical administration to warm-blooded animals in accordance with the present invention.

"Active ingredient" (A.I.) as used throughout these examples relates to a compound of formula (I), a pharmaceutically acceptable acid addition salt or a stereochemically isomeric form thereof.

Example 6 : ORAL DROPS

500 g of the A.I. was dissolved in 0.5 l of 2-hydroxypropanoic acid and 1.5 l of the polyethylene glycol at 60≈80 °C. After cooling to 30≈40 °C there were added 35 l of polyethylene glycol and the mixture was stirred well. Then there was added a solution of 1750 g of sodium saccharin in 2.5 l of purified water and while stirring there were added 2.5 l of cocoa flavor and polyethylene glycol q.s. to a volume of 50 l, providing an oral drop solution comprising 10 mg of the A.I. (per ml). The resulting solution was filled into suitable containers.

Example 7 : ORAL SOLUTION

9 g of methyl 4-hydroxybenzoate and 1 part of propyl 4-hydroxy-benzoate were dissolved in 4 l of boiling purified water. In 3 l of this solution were dissolved first 10 g of 2,3-dihydroxybutanedioic acid and thereafter 20 g of the A.I. The latter solution was combined with the remaining part of the former solution and 12 l 1,2,3-propane-triol and 3 l of sorbitol 70% solution were added thereto. 40 g of sodium saccharin were dissolved in 0.5 l of water and 2 ml of raspberry and 2 ml of gooseberry essence were added. The latter solution was combined with the former, water was added q.s. to a volume of 20 l providing an oral solution comprising 5 mg of the A.I. per teaspoonful (5 ml). The resulting solution was filled in suitable containers.

Example 8 : CAPSULES

20 g of the A.I., 6 g sodium lauryl sulfate, 56 g starch, 56 g lactose, 0.8 g colloidal silicon dioxide, and 1.2 g magnesium stearate were vigorously stirred together. The resulting mixture was subsequently filled into 1000 suitable hardened gelatin capsules, each comprising 20 mg of the A.I.

Example 9 : FILM-COATED TABLETS

Preparation of tablet core

A mixture of 100 g of the A.I., 570 g lactose and 200 g starch was mixed well and thereafter humidified with a solution of 5 g sodium dodecyl sulfate and 10 g polyvinylpyrrolidone (Kollidon-K 90®) in about 200 ml of water. The wet powder mixture was sieved, dried and sieved again. Then there was added 100 g microcrystalline cellulose (Avicel®) and 15 g hydrogenated vegetable oil (Sterotex®). The whole was mixed well and compressed into tablets, giving 10.000 tablets, each comprising 10 mg of the active ingredient.

Coating

To a solution of 10 g methyl cellulose (Methocel 60 HG®) in 75 ml of denaturated ethanol there was added a solution of 5 g of ethyl cellulose (Ethocel 22 cps ®) in 150 ml of dichloromethane. Then there were added 75 ml of dichloromethane and 2.5 ml 1,2,3-propane-triol. 10 g of polyethylene glycol was molten and dissolved in 75 ml of dichloromethane. The latter solution was added to the former and then there were added 2.5 g of magnesium octadecanoate, 5 g of polyvinylpyrrolidone and 30 ml of concentrated colour suspension (Opaspray K-1-2109®) and the whole was homogenated. The tablet cores were coated with the thus obtained mixture in a coating apparatus.

Example 10 : INJECTABLE SOLUTION

1.8 g methyl 4-hydroxybenzoate and 0.2 g propyl 4-hydroxybenzoate were dissolved in about 0.5 l of boiling water for injection. After cooling to about 50 °C there were added while stirring 4 g lactic acid, 0.05 g propylene glycol and 4 g of the A.I..The solution was cooled to room temperature and supplemented with water for injection q.s. ad 1 l volume, giving a solution of 4 mg A.I. per ml. The solution was sterilized by filtration (U.S.P. XVII p. 811) and filled in sterile containers.

Example 11 : SUPPOSITORIES

3 g A.I. was dissolved in a solution of 3 g 2,3-dihydroxybutane-dioic acid in 25 ml polyethylene glycol 400. 12 g surfactant (SPAN(®) and triglycerides (Witepsol 555®) q.s. ad 300 g were molten together. The latter mixture was mixed well with the former solution. The thus obtained mixture was poured into moulds at a temperature of 37-38 °C to form 100 suppositories each containing 30 mg of the active ingredient.

Example 12 : 2% CREAM

75 mg of stearyl alcohol, 2 mg of cetyl alcohol, 20 mg of sorbitan monostearate and 10 mg of isopropyl myristate are introduced into a doublewall jacketed vessel and heated until the mixture has completely molten. This mixture is added to a separately prepared mixture of purified water, 200 mg of propylene glycol and 15 mg of polysorbate 60 having a temperature of 70 to 75 °C while using a homogenizer for liquids. The resulting emulsion is allowed to cool to below 25 °C while continuously mixing. A solution of 20 mg of active ingredient of formula (I) or (II), 1 mg of polysorbate 80 and purified water and a solution of 2 mg of sodium sulfite anhydrous in purified water are next added to the emulsion while continuously mixing. The cream (1 g) is homogenized and filled into suitable tubes.

Example 13 : 2% TOPICAL GEL

To a solution of 200 mg of hydroxypropyl $\beta$-cyclodextrine in purified water is added 20 mg of active ingredient of formula (I) while stirring. Hydrochloric acid is added until complete solution and then sodium hydroxide added until pH 6.0. This solution is added to a dispersion of 10 mg of carrageenan PJ in 50 mg of propylene glycol while mixing. While mixing slowly the mixture is heated to 50 °C and allowed to cool to about 35 °C whereupon 50 mg of ethyl alcohol 95% is added. The rest of the purified water is added q.s. ad 1 g and the mixture is mixed to homogenous.

Example 14 : 2% TOPICAL CREAM

To a solution of 200 mg of hydroxypropyl $\beta$-cyclodextrine in purified water is added 20 mg of active ingredient of formula (I) while stirring. Hydrochloric acid is added until complete solution and next sodium hydroxide is added until pH 6.0. While stirring, 50 mg of glycerol and 35 mg of polysorbate 60 are added and the mixture is heated to 70 °C. The resulting mixture is added to a mixture of 100 mg of mineral oil, 20 mg of stearyl alcohol, 20 mg of cetyl alcohol, 20 mg of glycerol monostearate and 15 mg of sorbate 60 having a temperature of 70 °C while mixing slowly. After cooling down to below 25 °C, the rest of the purified water is added q.s. ad 1 g and the mixture is mixed to homogenous.

Example 15 : 2% LIPOSOME FORMULATION

A mixture of 2 g of active ingredient of formula (I) microfine, 20 g of phosphatidyl choline, 5 g of cholesterol and 10 g of ethyl alcohol is stirred and heated at 55-60 ° C until complete solution and is added to a solution of 0.2 g of methyl paraben, 0.02 g of propyl paraben, 0.15 g of disodium edetate and 0.3 g of sodium chloride in purified water while homogenizing. 1.5 g of hydroxypropylmethylcellulose in purified water is added ad, 100 g and the mixing is continued until swelling is complete.

Example 16 : 2% LIPOSOME FORMULATION

A mixture of 10 g of phosphatidyl choline and 1 g of cholesterol in 7.5 g of ethyl alcohol is stirred and heated at 40 ° C until complete solution. 2 g of active ingredient of formula (I) microfine is dissolved in purified water by mixing while heating at 40 ° C. The alcoholic solution is added slowly to the aqueous solution while homogenizing during 10 minutes. 1.5 g of hydroxypropylmethylcellulose in purified water is added while mixing until swelling is complete. The resulting solution is adjusted to pH 5.0 with sodium hydroxide 1 N and diluted with the rest of the purified water ad 100 g.

## Claims

1. The use for the manufacture of a medicament for treating subjects suffering from disorders which are characterized by an increased proliferation and/or abnormal differentiation of normal, preneoplastic or neoplastic epithelial cells, the growth of which is sensitive to the actions of retinoids, of a compound of formula

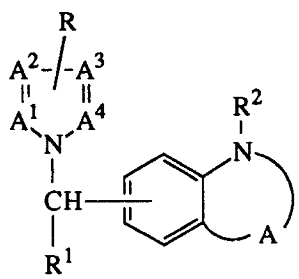

(I)

a pharmaceutically acceptable acid addition salt thereof or a stereochemically isomeric form thereof, wherein

$-A^1=A^2-A^3=A^4-$ is a bivalent radical having the formula

$-CH=N-CH=CH-$ (x);

$-CH=N-CH=N-$ (y);

or

$-CH=N-N=CH-$ (z);

R is hydrogen or $C_{1-6}$alkyl;

$R^1$ is hydrogen; $C_{1-10}$alkyl; $C_{3-7}$cycloalkyl; $Ar^1$ or $Ar^1$-$C_{1-6}$alkyl;

$R^2$ is hydrogen; $C_{3-7}$cycloalkyl; $Ar^1$; $C_{1-10}$alkyl; $C_{1-6}$alkyl substituted with $Ar^1$ or $C_{3-7}$cycloalkyl; hydroxy; $C_{1-10}$alkyloxy; $C_{1-6}$alkyloxy substituted with $Ar^1$ or $C_{3-7}$cycloalkyl; $C_{3-6}$alkenyloxy optionally substituted with $Ar^2$; $C_{3-6}$alkynyloxy optionally substituted with $Ar^2$; or $Ar^1$-oxy;

A is a bivalent radical having the formula

$-CR^3=N-$ (a)

or

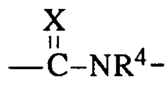 (b),

wherein the carbon atom in the bivalent radical (a) and (b) is connected to $-NR^2$;

said $R^3$ being hydrogen; halo; $C_{1-4}$alkyl substituted with up to 4 halo atoms; $C_{3-7}$cycloalkyl; $Ar^1$; quinolinyl; indolinyl; $C_{1-10}$alkyl; $C_{1-6}$alkyl substituted with $Ar^1$, $C_{3-7}$cycloalkyl, quinolinyl, indolinyl or hydroxy; $C_{1-10}$alkyloxy; $C_{1-6}$alkyloxy substituted with $Ar^1$ or $C_{3-7}$cycloalkyl; $C_{2-6}$alkenyl optionally substituted with $Ar^1$; $Ar^2$-oxy; $C_{1-6}$alkyloxycarbonyl; carboxyl; $C_{1-6}$alkylcarbonyl; $Ar^1$-carbonyl or $Ar^1$-(CHOH)-;

said X being O or S;

said $R^4$ being hydrogen, $C_{1-6}$alkyl or $Ar^2$-$C_{1-6}$alkyl;

wherein $Ar^1$ is phenyl, substituted phenyl, pyridinyl, aminopyridinyl, imidazolyl, thienyl, halothienyl, furanyl, halofuranyl or thiazolyl; and $Ar^2$ is phenyl or substituted phenyl; said substituted phenyl in $Ar^1$ and $Ar^2$ being phenyl substituted with 1, 2 or 3 substituents each independently selected from halo, hydroxy, trifluoromethyl, $C_{1-6}$alkyl, $C_{1-6}$alkyloxy, cyano, amino, mono- and di($C_{1-6}$alkyl)amino, nitro, carboxyl, formyl and $C_{1-6}$alkyloxycarbonyl.

2. The use of a compound according to claim 1 wherein in the compound of formula (I) the 1H-azol-1-ylmethyl moiety is substituted on either the 5 or 6 position of the benzimidazole ring; R is hydrogen; $R^1$ is hydrogen; $C_{1-6}$alkyl; $C_{3-7}$cycloalkyl; phenyl; substituted phenyl; thienyl or furanyl optionally substituted with halo; $R^2$ is hydrogen; $C_{3-7}$cycloalkyl; phenyl; substituted phenyl; pyridinyl; $C_{1-6}$alkyl optionally monosubstituted with phenyl, $C_{3-7}$cycloalkyl, pyridinyl or thienyl; hydroxyl, $C_{1-6}$alkyloxy optionally monosubstituted with phenyl, pyridinyl, thienyl or $C_{3-6}$cycloalkyl; $C_{3-6}$alkenyloxy optionally monosubstituted with phenyl; or $C_{3-6}$alkynyloxy; $R^3$ is hydrogen; $C_{1-4}$alkyl substituted with up to 4 halo atoms; $C_{3-7}$cycloalkyl; phenyl; substituted phenyl; imidazolyl; thiazolyl; thienyl; furanyl; quinolinyl; pyridinyl optionally substituted with amino, $C_{1-10}$alkyl; $C_{1-5}$alkyl optionally monosubstituted with phenyl, pyridinyl, imidazolyl, thienyl, indolyl or hydroxyl; $C_{1-4}$alkyloxy optionally monosubstituted with phenyl; $C_{2-6}$alkenyl optionally monosubstituted with pyridinyl, furanyl, imidazolyl or phenyl; carboxyl; $C_{1-4}$alkyloxycarbonyl; phenylcarbonyl; or hydroxy and phenylmethyl; and $R^4$ is hydrogen or phenyl$C_{1-4}$alkyl.

3. The use of a compound according to claim 2 wherein $R^1$ is hydrogen, $C_{1-6}$alkyl, phenyl, substituted phenyl, thienyl or furanyl; $R^2$ is hydrogen, $C_{1-6}$alkyl, or $C_{1-4}$alkyl substituted with phenyl; $R^3$ is hydrogen, $C_{1-6}$alkyl, phenyl, pyridinyl, $C_{1-6}$alkyl, $C_{1-6}$alkyl monosubstituted with phenyl or $C_{2-6}$alkenyl optionally monosubstituted with furanyl or phenyl; and $R^4$ is hydrogen.

4. The use of a compound according to claim 3 wherein $R^1$ is $C_{1-4}$alkyl, phenyl, phenyl substituted with one or two halo, $C_{1-4}$alkyl or $C_{1-4}$alkyloxy substituents, or thienyl; $R^2$ is hydrogen or $C_{1-4}$alkyl; and $R^3$ is hydrogen or $C_{1-4}$alkyl.

5. The use of a compound according to claim 1 wherein the compound is 5-[(1H-imidazol-1-yl)-phenylmethyl]-1H-benzimidazole, (±)-5-[(1H-imidazol-1-yl)phenylmethyl]-2-methyl-1H-benzimidazole, 5-[(1H-imidazol-1-yl)phenylmethyl]-1-methyl-1H-benzimidazole, 5-[1-(1H-imidazole-1-yl)-2-methylpropyl]-2-methyl-1H-benzimidazole, 5-[(3-chlorophenyl) (1H-imidazol-1-yl)methyl]-1H-benzimidazole or (±)-5-[-(1H-imidazol-1-yl)phenylmethyl]-2-methyl-1H-benzimidazole, a pharmaceutically acceptable acid addition salt or a possible stereoisomer thereof.

6. A chemical compound having the formula (I) as defined in claim 1, provided that R is other than hydrogen when $-A^1=A^2-A^3=A^4-$ is a bivalent radical of formula (x).

7. A chemical compound according to claim 6 wherein $-A^1=A^2-A^3=A^4-$ is a bivalent radical of formula (x) or (y).

8. A chemical compound according to claim 7 wherein $R^1$ is phenyl or halophenyl; $R^2$ is hydrogen or $C_{1-4}$alkyl; and $R^3$ is hydrogen or $C_{1-4}$alkyl.

**9.** A pharmaceutical composition comprising a pharmaceutically acceptable carrier and as active ingredient a compound of formula (I) as defined in any of claims 6 to 8.

**10.** A compound of formula (I) as defined in any of claims 6 to 8 for use as a medicine.

**11.** The use of a compound of formula (I) as defined in any of claims 1 to 5 for the manufacture of a medicament for controling the rate of growth and differentiation of normal, preneoplastic and neoplastic epithelial cells, the growth and differentiation of which is sensitive to the actions of retinoids.

**12.** The use of a compound of formula (I) as defined in any of claims 1 to 5 for the manufacture of a medicament for reducing or curing acne.

**13.** The use of a chemical compound as defined in any of claims 1 to 5 for the manufacture of a medicament for inhibiting the metabolism of retinoic acid in mammals.

**14.** The use of a chemical compound as defined in any of claims 1 to 5 for the manufacture of a medicament for treating psoriasis.

**15.** A process for preparing a compound of formula (I) as claimed in any of claims 6 to 8, by

a) N-alkylating an azole of formula (III) or an alkali metal salt thereof

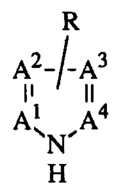

(III)

with a benzimidazole of formula

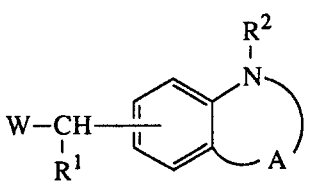

(IV)

wherein W represents a reactive leaving group;

b) endo-N-alkylating an azole of formula

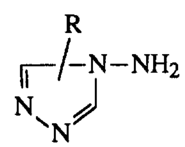

(III-y)

wherein R is as defined under formula (I) with a benzimidazole of formula (IV) and subsequently deaminating the thus obtained triazolium salt,

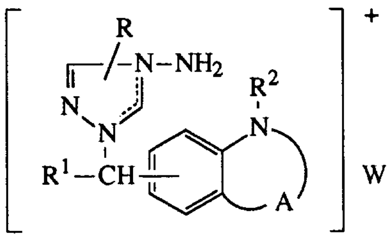

thus preparing a compound of formula

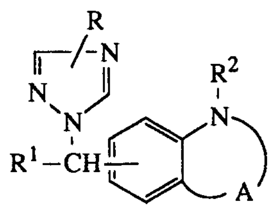

(I-y) ;

c) reacting a compound of formula

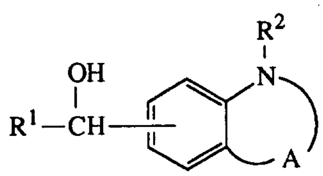

(VII)

with a reagent of formula

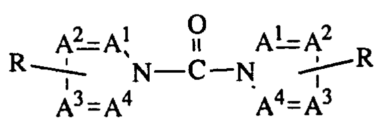

(IX)

in a suitable solvent;

d) reductively alkylating a ketone or aldehyde of formula

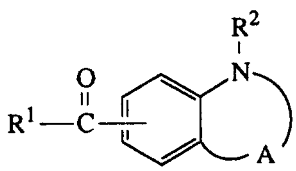

(X)

with an azole of formula

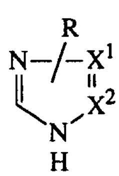

(III)

by stirring and heating the reagents in formic acid or formamides in the presence of an acid catalyst;
e) heating an intermediate of formula

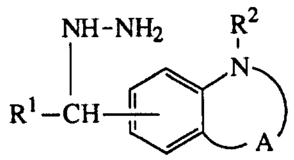

(XVI)

with s-triazine, thus obtaining a compound of formula

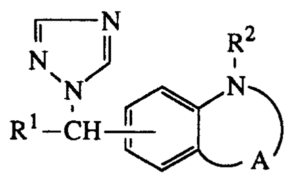

(I-y)

or optionally convening the compounds of formula (I) into each other following art-known grouptransformation procedures, and, if desired, converting the compounds of formula (I) into a therapeutically active non-toxic acid-addition salt form by treatment with an appropriate acid or, conversely, converting the acid-addition salt into the free base form with alkali; and/or preparing stereochemically isomeric forms thereof.

**Patentansprüche**

**1.** Verwendung einer Verbindung der Formel

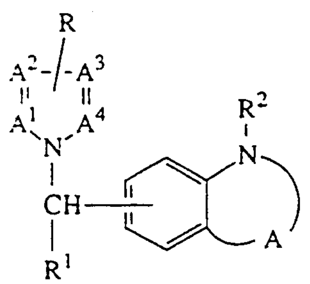

(I) ,

eines pharmazeutisch annehmbaren Säureadditionssalzes hievon oder einer stereochemisch isomeren Form hievon, worin

$-A^1=A^2-A^3=A^4-$ einen Zweiwertigen Rest mit der Formel

-CH = N-CH = CH- (x);

-CH = N-CH = N- (y);

oder

-CH = N-N = CH- (z)

bedeutet;

R Wasserstoff oder $C_{1-6}$-Alkyl darstellt;

$R^1$ für Wasserstoff; $C_{1-10}$-Alkyl; $C_{3-7}$-Cycloalkyl; $Ar^1$ oder $Ar^1$-$C_{1-6}$-Alkyl steht;

$R^2$ Wasserstoff; $C_{3-7}$-Cycloalkyl; $Ar^1$; $C_{1-10}$-Alkyl; durch $Ar^1$ oder $C_{3-7}$-Cycloalkyl substituiertes $C_{1-6}$-Alkyl; Hydroxy; $C_{1-10}$-Alkyloxy; durch $Ar^1$ oder $C_{3-7}$-Cycloalkyl substituiertes $C_{1-6}$-Alkyloxy;

$C_{3-6}$-Alkenyloxy, das gegebenenfalls durch $Ar^2$ substituiert ist; $C_{3-6}$-Alkinyloxy, das gegebenenfalls durch $Ar^2$ substituiert ist; oder $Ar^1$-Oxy bedeutet;

A für einen zweiwertigen Rest mit der Formel

$-CR^3=N-$ (a)

oder

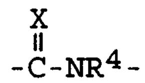 (b)

steht, worin das Kohlenstoffatom in dem zweiwertigen Rest (a) und (b) an $-NR^2$ gebunden ist;

wobei der genannte Rest $R^3$ Wasserstoff; Halogen; durch bis zu 4 Halogenatome substituiertes $C_{1-4}$-Alkyl; $C_{3-7}$-Cycloalkyl; $Ar^1$; Chinolinyl; Indolinyl; $C_{1-10}$-Alkyl; durch $Ar^1$, $C_{3-7}$-Cycloalkyl, Chinolinyl, Indolinyl oder Hydroxy substituiertes $C_{1-6}$-Alkyl; $C_{1-10}$-Alkyloxy; durch $Ar^1$ oder $C_{3-7}$-Cycloalkyl substituiertes $C_{1-6}$-Alkyloxy; gegebenenfalls durch $Ar^1$ substituiertes $C_{2-6}$-Alkenyl; $Ar^2$-Oxy; $C_{1-6}$-Alkyloxycarbonyl; Carboxyl; $C_{1-6}$-Alkylcarbonyl; $Ar^1$-Carbonyl oder $Ar^1$-(CHOH) - bedeutet;

wobei der Rest X für O oder S steht;

wobei der Rest $R^4$ Wasserstoff, $C_{1-6}$-Alkyl oder $Ar^2$-$C_{1-6}$-Alkyl darstellt;

worin $Ar^1$ für Phenyl, substituiertes Phenyl, Pyridinyl, Aminopyridinyl, Imidazolyl, Thienyl, Halogenthienyl, Furanyl, Halogenfuranyl oder Thiazolyl steht; und worin $Ar^2$ Phenyl oder substituiertes Phenyl darstellt; wobei das substituierte Phenyl in $Ar^1$ und $Ar^2$ ein durch 1, 2 oder 3 Substituenten substituiertes Phenyl bedeutet, wobei die Substituenten jeweils unabhängig unter Halogen, Hydroxy, Trifluormethyl, $C_{1-6}$-Alky1, $C_{1-6}$-Alkyloxy, Cyano, Amino, Mono- und Di($C_{1-6}$-alkyl)amino, Nitro, Carboxyl, Formyl und $C_{1-6}$-Alkyloxycarbonyl ausgewählt sind,

zur Herstellung eines Medikaments zur Behandlung von Patienten, die unter Störungen leiden, die durch eine erhöhte Proliferation und/oder abnormale Differenzierung von normalen, präneoplastischen oder neoplastischen Epithelialzellen, deren Wachstum gegenüber den Wirkungen von Retinoiden empfindlich ist, gekennzeichnet sind.

**2.** Verwendung einer Verbindung nach Anspruch 1, wobei in der Verbindung der Formel (I) der 1H-Azol-1-ylmethyl-Rest als Substituent an der 5- oder 6-Stellung des Benzimidazolringes vorliegt; R Wasserstoff bedeutet; $R^1$ für Wasserstoff; $C_{1-6}$-Alkyl; $C_{3-7}$-Cycloalkyl; Phenyl; substituiertes Phenyl; Thienyl oder Furanyl, gegebenenfalls durch Halogen substituiert, steht; $R^2$ Wasserstoff; $C_{3-7}$-Cycloalkyl; Phenyl; substituiertes Phenyl; Pyridinyl; gegebenenfalls durch Phenyl, $C_{3-7}$-Cycloalkyl, Pyridinyl oder Thienyl einfach substituiertes $C_{1-6}$-Alkyl; Hydroxyl; gegebenenfalls durch Phenyl, Pyridinyl, Thienyl oder $C_{3-6}$-Cycloalkyleinfach substituiertes $C_{1-6}$-Alkyloxy; gegebenenfalls durch Phenyl einfach substituiertes $C_{3-6}$-Alkenyloxy; oder $C_{3-6}$-Alkinyloxy darstellt; $R^3$ für Wasserstoff; durch bis zu 4 Halogenatome substituiertes $C_{1-4}$-Alkyl; $C_{3-7}$-Cycloalkyl; Phenyl; substituiertes Phenyl; Imidazolyl; Thiazolyl; Thienyl; Furanyl; Chinolinyl; gegebenenfalls durch Amino, $C_{1-10}$-Alkyl substituiertes Pyridinyl; gegebenenfalls durch Phenyl, Pyridinyl, Imidazolyl, Thienyl, Indolyl oder Hydroxyl einfach substituiertes $C_{1-5}$-Alkyl; gegebenenfalls durch Phenyl einfach substituiertes $C_{1-4}$-Alkyloxy; gegebenenfalls durch Pyridinyl, Furanyl, Imidazolyl oder Phenyl einfach substituiertes $C_{2-6}$-Alkenyl; Carboxyl; $C_{1-4}$-Alkyloxycarbonyl; Phenylcarbonyl; oder Hydroxy und Phenylmethyl steht; und $R^4$ Wasserstoff oder Phenyl-$C_{1-4}$-alkyl bedeutet.

**3.** Verwendung einer Verbindung nach Anspruch 2, worin $R^1$ Wasserstoff, $C_{1-6}$-Alkyl, Phenyl, substituiertes Phenyl, Thienyl oder Furanyl bedeutet; $R^2$ für Wasserstoff, $C_{1-6}$-Alkyl oder durch Phenyl substituiertes $C_{1-4}$-Alkyl steht; $R^3$ Wasserstoff, $C_{1-6}$-Alkyl, Phenyl, Pyridinyl, $C_{1-6}$-Alkyl, durch Phenyl monosubstituiertes $C_{1-6}$-Alkyl oder gegebenenfalls durch Furanyl oder Phenyl monosubstituiertes $C_{2-6}$-Alkenyl darstellt; und $R^4$ Wasserstoff bedeutet.

**4.** Verwendung einer Verbindung nach Anspruch 3, worin $R^1$ für $C_{1-4}$-Alkyl, Phenyl, durch ein oder zwei Halogen-, $C_{1-4}$-Alkyl- oder $C_{1-4}$-Alkyloxysubstituenten substituiertes Phenyl oder Thienyl steht; $R^2$ Wasserstoff oder $C_{1-4}$-Alkyl bedeutet; und $R^3$ Wasserstoff oder $C_{1-4}$-Alkyl darstellt.

**5.** Verwendung einer Verbindung nach Anspruch 1, worin die Verbindung 5-[(1H-Imidazol-1-yl)-phenylmethyl]-1H-benzimidazol, (± )-5-[(1H-Imidazol-1-yl)phenylmethyl]-2-methyl-1H-benz-imidazol, 5-[-(1H-Imidazol-1-yl)phenylmethyl]-1-methyl-1H-benz-imidazol, 5-[1-(1H-Imidazol-1-yl)-2-methylpropyl]-2-methyl-1H-benzimidazol, 5-[(3-Chlorphenyl)-(1H-imidazol-1-yl)methyl]-1H-benzimidazol oder (±)-5-[(1H-Imidazol-1-yl)phenylmethyl]-2-methyl-1H-benzimidazol, ein pharmazeutisch annehmbares Säureadditionssalz oder ein mögliches Stereoisomer hievon ist.

**6.** Chemische Verbindung mit der Formel (I), wie in Anspruch 1 definiert, mit der Maßgabe, daß R eine andere Bedeutung als Wasserstoff hat, wenn $-A^1=A^2-A^3=A^4-$ einen zweiwertigen Rest der Formel (x) darstellt.

**7.** Chemische Verbindung nach Anspruch 6, worin $-A^1=A^2-A^3=A^4-$einen zweiwertigen Rest der Formel (x) oder (y) darstellt.

**8.** Chemische Verbindung nach Anspruch 7, worin $R^1$ für Phenyl oder Halogenphenyl steht; $R^2$ Wasserstoff oder $C_{1-4}$-Alkyl bedeutet; und $R^3$ Wasserstoff oder $C_{1-4}$-Alkyl darstellt.

**9.** Pharmazeutische Zusammensetzung, umfassend einen pharmazeutisch annehmbaren Träger und als wirksamen Bestandteil eine Verbindung der Formel (I), wie in einem der Ansprüche 6 bis 8 definiert.

**10.** Verbindung der Formel (I), wie in einem der Ansprüche 6 bis 8 definiert, zur Verwendung als eine Medizin.

**11.** Verwendung einer Verbindung der Formel (I), wie in einem der Ansprüche 1 bis 5 definiert, zur Herstellung eines Medikaments für die Regelung der Wachstumsgeschwindigkeit und Differenzierung von normalen, präneoplastischen und neoplastischen Epithelialzellen, deren Wachstum und Differenzierung gegenüber den Wirkungen von Retinoiden empfindlich sind.

**12.** Verwendung einer Verbindung der Formel (I), wie in einem der Ansprüche 1 bis 5 definiert, für die Herstellung eines Medikaments zur Verminderung oder Heilung von Akne.

**13.** Verwendung einer chemischen Verbindung, wie in einem der Ansprüche 1 bis 5 definiert, zur Herstellung eines Medikaments zur Inhibierung des Metabolismus von Retinoesäure in Säugetieren.

**14.** Verwendung einer chemischen Verbindung, wie in einem der Ansprüche 1 bis 5 definiert, zur Herstellung eines Medikaments zur Behandlung von Psoriasis.

**15.** Verfahren zur Herstellung einer Verbindung der Formel (I), wie in einem der Ansprüche 6 bis 8 definiert, durch

a) N-Alkylieren eines Azols der Formel (III) oder eines Alkalimetallsalzes hievon

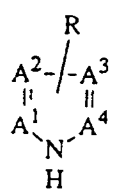 (III)

mit einem Benzimidazol der Formel

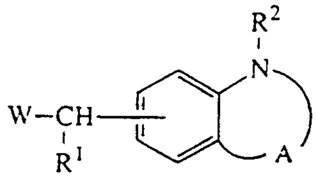 (IV) ,

worin W eine reaktionsfähige Leaving-Gruppe bedeutet;
b) Endo-N-alkylieren eines Azols der Formel

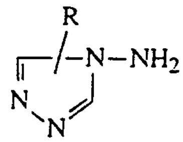

(III-y) ,

worin R wie unter Formel (I) definiert ist, mit einem Benzimidazol der Formel (IV) und anschließendes Deaminieren des so erhaltenen Triazoliumsalzes

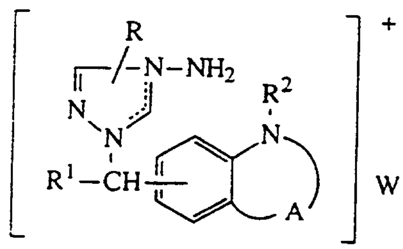

unter Ausbildung einer Verbindung der Formel

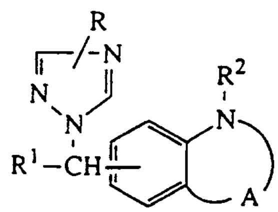

(I-y) ;

c) Umsetzen einer Verbindung der Formel

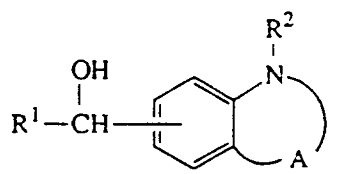

(VII)

mit einem Reagens der Formel

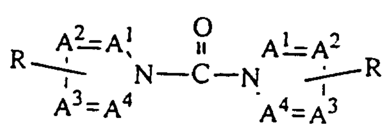

(IX)

in einem geeigneten Lösungsmittel;

d) reduktives Alkylieren eines Ketons oder Aldehyds der Formel

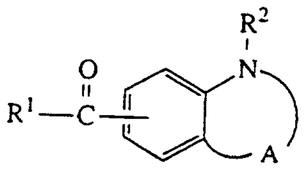

(X)

mit einem Azol der Formel

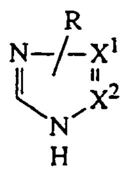

(III)

durch Rühren und Erwärmen der Reagenzien in Ameisensäure oder in Formamiden in Anwesenheit eines sauren Katalysators;
e) Erhitzen eines Zwischenprodukts der Formel

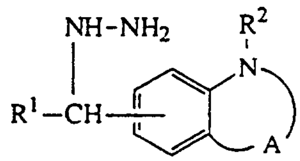

(XVI)

mit s-Triazin unter Ausbildung einer Verbindung der Formel

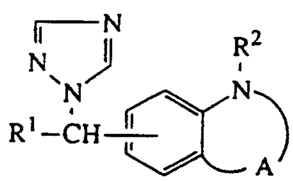

(I-y)

oder gewünschtenfalls Überführen der Verbindungen der Formel (I) ineinander nach bekannten Gruppentransformationsverfahren und, falls gewünscht, Überführen der Verbindungen der Formel (I) in eine therapeutisch wirksame nicht-toxische Säureadditionssalzform durch Behandlung mit einer entsprechenden Säure, oder umgekehrt Überführen des Säureadditionssalzes in die freie Basenform mit Alkali; und/oder Bereiten stereochemisch isomerer Formen hievon.

**Revendications**

**1.** Utilisation d'un composé de formule

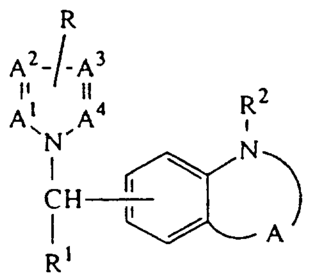

(I)

d'un sel d'addition avec un acide pharmaceutiquement acceptable de celui-ci ou d'une forme stéréochimiquement isomère de celui-ci,
formule dans laquelle
$-A^1=A^2-A^3=A^4-$ est un radical bivalent de formule

$-CH=N-CH=CH-$ (x),

$-CH=N-CH=N-$ (y)

ou

$-CH=N-N=CH-$ (z);

R est un atome d'hydrogène ou un groupe alkyle en $C_{1-6}$;
$R^1$ est un atome d'hydrogène ou un groupe alkyle en $C_{1-10}$, cycloalkyle en $C_{3-7}$, $Ar^1$ ou $Ar^1$-alkyle($C_{1-6}$);
$R^2$ est un atome d'hydrogène ou un groupe cycloalkyle en $C_{3-7}$, $Ar^1$, alkyle en $C_{1-10}$, alkyle en $C_{1-6}$ substitué par un radical $Ar^1$ ou cycloalkyle en $C_{3-7}$; le groupe hydroxy; un groupe alkyloxy en $C_{1-10}$, alkyloxy en $C_{1-6}$ substitué par un radical $Ar^1$ ou cycloalkyle en $C_{3-7}$; un groupe alcényloxy en $C_{3-6}$ éventuellement substitué par un substituant $Ar^2$; un groupe alcynyloxy en $C_{3-6}$ éventuellement substitué par un substituant $Ar^2$; ou un groupe $Ar^1$-oxy;
A est un radical bivalent de formule

$-CR^3=N-$ (a)

ou

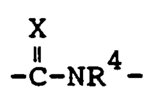

(b),

l'atome de carbone dans les radicaux bivalents (a) et (b) étant lié à $-NR^2$;
ledit radical $R^3$ étant un atome d'hydrogène ou d'halogène ou un groupe alkyle en $C_{1-4}$ substitué par jusqu'à 4 atomes d'halogène: un groupe cycloalkyle en $C_{3-7}$, $Ar^1$, quinolinyle, indolinyle, alkyle en $C_{1-10}$, alkyle en $C_{1-6}$ substitué par un substituant $Ar^1$, cycloalkyle en $C_{3-7}$, quinolinyle, indolinyle ou hydroxy: un groupe alkyloxy en $C_{1-10}$, alkyloxy en $C_{1-6}$ substitué par un substituant $Ar^1$ ou cycloalkyle en $C_{3-7}$; un groupe alcényle en $C_{2-6}$ éventuellement substitué par un substituant $Ar^1$, $Ar^2$-oxy, alkyloxy($C_{1-6}$)carbonyle, carboxy, alkyl($C_{1-6}$)carbonyle, $Ar^1$-carbonyle ou $Ar^1$-(CHOH)-;
ledit radical X étant O ou S:
ledit radical $R^4$ étant un atome d'hydrogène ou un groupe alkyle en $C_{1-6}$ ou $Ar^2$-alkyle($C_{1-6}$);
$Ar^1$ étant un radical phényle, phényle substitué, pyridinyle, aminopyridinyle, imidazolyle,

thiényle, halogénothiényle, furannyle, halogénofurannyle ou thiazolyle: et $Ar^2$ étant un radical phényle ou phényle substitué; ledit radical phényle substitué dans $Ar^1$ et $Ar^2$ étant un radical phényle substitué par 1, 2 ou 3 substituants choisis chacun indépendamment parmi des atomes d'halogène et des groupes hydroxy, trifluorométhyle, alkyle en $C_{1-6}$, alkyloxy en $C_{1-6}$, cyano, amino, mono- et dialkyl($C_{1-6}$)amino,nitro, carboxy, formyle et alkyloxy($C_{1-6}$)-carbonyle,

pour la fabrication d'un médicament pour le traitement de sujets souffrant de troubles qui sont caractérisés par une prolifération accrue et/ou une différenciation anormale de cellules épithéliales normales, prénéoplasiques ou néoplasiques, dont la croissance est sensible à l'action de rétinoïdes.

2. Utilisation d'un composé selon la revendication 1, caractérisée en ce que, dans le composé de formule (I), le fragment 1H-azol-1-ylméthyle est porté sur la position 5 ou 6 du cycle benzimidazole; R est un atome d'hydrogène; $R^1$ est un atome d'hydrogène ou un groupe alkyle en $C_{1-6}$, cycloalkyle en $C_{3-7}$, phényle, phényle substitué, thiényle ou furannyle éventuellement substitués par un substituant halogène; $R^2$ est un atome d'hydrogène ou un radical cycloalkyle en $C_{3-7}$, phényle, phényle substitué, pyridinyle, alkyle en $C_{1-6}$ éventuellement monosubstitué par un groupe phényle, cycloalkyle en $C_{3-7}$, pyridinyle ou thiényle; le radical hydroxy, un radical alkyloxy en $C_{1-6}$ éventuellement monosubstitué par un groupe phényle, pyridinyle, thiényle ou cycloalkyle en $C_{3-6}$; un radical alcényloxy en $C_{3-6}$ éventuellement monosubstitué par le groupe phényle; ou un radical alcynyloxy en $C_{3-6}$; $R^3$ est un atome d'hydrogène ou un radical alkyle en $C_{1-4}$ substitué par jusqu'à 4 atomes d'halogène; un radical cycloalkyle en $C_{3-7}$, phényle, phényle substitué, imidazolyle, thiazolyle, thiényle, furannyle, quinolinyle, pyridinyle éventuellement substitué par le groupe amino: un radical alkyle en $C_{1-10}$, alkyle en $C_{1-5}$ éventuellement monosubstitué par le groupe phényle, pyridinyle, imidazolyle, thiényle, indolyle, ou hydroxy; un radical alkyloxy en $C_{1-4}$ éventuellement monosubstitué par le groupe phényle; un radical alcényle en $C_{2-6}$ éventuellement monosubstitué par le groupe pyridinyle, furannyle, imidazolyle ou phényle; un radical carboxy, alkyloxy($C_{1-4}$)carbonyle, phénylcarbonyle ou hydroxy et phénylméthyle; $R^4$ est un atome d'hydrogène ou un radical phényl-alkyle($C_{1-4}$).

3. Utilisation d'un composé selon la revendication 2, dans lequel $R^1$ est un atome d'hydrogène ou un groupe alkyle en $C_{1-6}$, phényle, phényle substitué, thiényle ou furannyle; $R^2$ et un atome d'hydrogène ou un groupe alkyle en $C_{1-6}$ ou un groupe alkyle en $C_{1-4}$ substitué par le radical phényle; $R^3$ est un atome d'hydrogène ou un groupe alkyle en $C_{1-6}$, phényle, pyridinyle, alkyle en $C_{1-6}$, alkyle en $C_{1-6}$ monosubstitué par le groupe phényle, ou un groupe alcényle en $C_{2-6}$ éventuellement monosubstitué par le radical furannyle ou phényle; et $R^4$ est un atome d'hydrogène.

4. Utilisation d'un composé selon la revendication 3, dans lequel $R^1$ est un groupe alkyle en $C_{1-4}$, phényle, phényle substitué par un ou deux substituants halogène, alkyle en $C_{1-4}$ ou alkyloxy en $C_{1-4}$, ou le groupe thiényle: $R^2$ est un atome d'hydrogène ou un groupe alkyle en $C_{1-4}$; et $R^3$ est un atome d'hydrogène ou un groupe alkyle en $C_{1-4}$.

5. Utilisation d'un composé selon la revendication 1, caractérisée en ce que le composé est le 5-[(1H-imidazol-1-yl)phénylméthyl]-1H-benzimidazole, le (±)-5-[(1H)-imidazol-1-yl)phénylméthyl]-2-méthyl-1H-benzimidazole, le 5-((1H-imidazol-1-yl)phénylméthyl]-1-méthyl-1H-benzimidazole, le 5-(1-(1H-imidazol-1-yl)-2-méthylpropyl]-2-méthyl-1H-benzimidazole, le 5-((3-chlorophényl)(1H-imidazol-1-yl)méthyl]-1H-benzimidazole ou le (±)-5-((1H-imidazol-1-yl)phénylméthyl)-2-méthyl-1H-benzimidazole, un sel d'addition avec un acide pharmaceutiquement acceptable de celui-ci ou un stéréoisomère possible de celui-ci.

6. Composé chimique de formule (I) tel que défini dans la revendication 1, étant entendu que R est différent d'un atome d'hydrogène lorsque $-A^1=A^2-A^3=A^4-$ est un radical bivalent de formule (x).

7. Composé chimique selon la revendication 6, dans lequel $-A^1=A^2-A^3=A^4-$ est un radical bivalent de formule (x) ou (y).

8. Composé chimique selon la revendication 7, dans lequel $R^1$ est un groupe phényle ou halogénophényle; $R^2$ est un atome d'hydrogène ou un groupe alkyle en $C_{1-4}$; et $R^3$ et un atome d'hydrogène ou un groupe alkyle en $C_{1-4}$.

**9.** Composition pharmaceutique comprenant un véhicule pharmaceutiquement acceptable et, en tant que composant actif, un composé de formule (I) tel que défini dans l'une quelconque des revendications 6 à 8.

**10.** Composé de formule (I) tel que défini dans l'une quelconque des revendications 6 à 8, pour utilisation en tant que médicament.

**11.** Utilisation d'un composé de formule (I) tel que défini dans l'une quelconque des revendications 1 à 5, pour la fabrication d'un médicament pour la régulation de la vitesse de croissance et de la différenciation de cellules épithéliales normales, prénéoplasiques et néoplasiques, dont la croissance et la différenciation sont sensibles à l'action de rétinoïdes.

**12.** Utilisation d'un composé de formule (I) tel que défini dans l'une quelconque des revendications 1 à 5, pour la fabrication d'un médicament pour la diminution ou la disparition de l'acné.

**13.** Utilisation d'un composé chimique tel que défini dans l'une quelconque des revendications 1 à 5, pour la fabrication d'un médicament pour l'inhibition du métabolisme de l'acide rétinoïque chez les mammifères.

**14.** Utilisation d'un composé chimique tel que défini dans l'une quelconque des revendications 1 à 5, pour la fabrication d'un médicament pour le traitement du psoriasis.

**15.** Procédé pour la préparation d'un composé de formule (I) selon l'une quelconque des revendications 6 à 8, par

a) N-alkylation d'un azole de formule (III) ou d'un sel alcalin de celui-ci

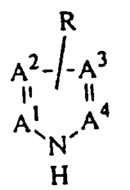

(III)

avec un benzimidazole de formule

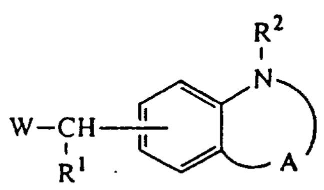

(IV)

dans laquelle W représente un groupe partant réactif;

b) endo-N-alkylation d'un azole de formule

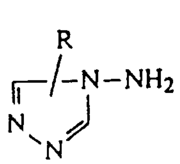

(III-y)

dans laquelle R est tel que défini à propos de la formule (I), avec un benzimidazole de formule (IV), et ensuite désamination du sel de thiazolium ainsi obtenu,

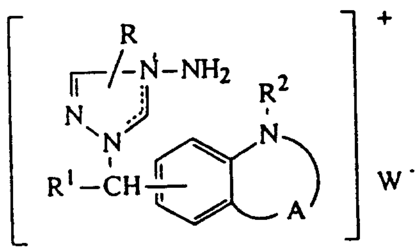

pour l'obtention d'un composé de formule

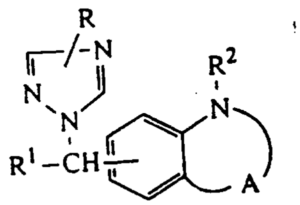

(I-y) ;

c) mise en réaction d'un composé de formule

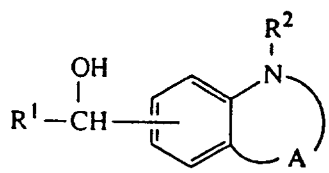

(VII)

avec un réactif de formule

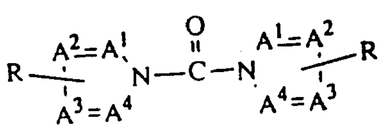

(IX)

dans un solvant approprié;
d) alkylation réductrice d'une cétone ou d'un aldéhyde de formule

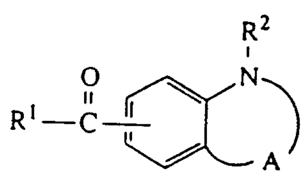

(X)

avec un azole de formule

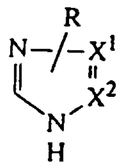

(III)

par agitation et chauffage des réactifs dans de l'acide formique ou des formamides, en présence d'un catalyseur acide;

e) chauffage d'un produit intermédiaire de formule

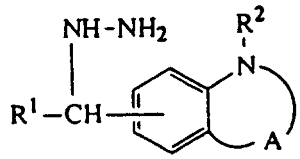

(XVI)

avec de la triazine-s, pour l'obtention d'un composé de formule

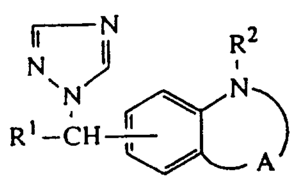

(I-y)

ou éventuellement conversion des composés de formule (I) entre eux, selon des méthodes connues de transformation de groupes, et, si on le désire, conversion des composés de formule (I) en une forme de sel d'addition avec un acide non toxique et thérapeutiquement actif, par traitement par un acide approprié, ou, inversement, conversion du sel d'addition avec un acide en la base libre avec un alcali; et/ou préparation de formes stéréochimiquement isomères de ce composé.